# EUROPÄISCHE PATENTSCHRIFT

(11) **EP 2 087 879 B1**
(45) Veröffentlichungstag und Bekanntmachung des Hinweises auf die Patenterteilung: **13.04.2011**
(21) Anmeldenummer: 09152324.1
(22) Anmeldetag: 06.02.2009
(51) Int. Cl.: A61K 8/35, A61Q 19/04

(54) **Hauttönungsmittel mit DHA auf Basis Wasser-Wachs-Öl**
Skin toning agent with DHA on a water-wax-oil basis
Moyen de teinture de la peau doté de DHA à base d'eau-cire-huile

(30) Priorität: 07.02.2008 DE 102008008473
(43) Veröffentlichungstag der Anmeldung: 12.08.2009
(73) Patentinhaber: Coty Prestige Lancaster Group GmbH, 55116 Mainz (DE)
(72) Erfinder: Golz-Berner, Karin, 98000 Monaco (MC); Zastrow, Leonhard, 98000 Monaco (MC)
(74) Vertreter: Gulde Hengelhaupt Ziebig & Schneider

(56) Entgegenhaltungen:
- EP-A- 1 473 026
- EP-A- 1 477 159
- WO-A-01/70186
- DE-A1- 19 603 018

## Beschreibung

Die Erfindung betrifft ein Hauttönungsmittel mit DHA auf Basis einer Formulierung mit Wasser, Wachs und Öl, die keinen Emulgator enthält.

Dihydroxyaceton (DHA) ist das bekannteste Hautbräunungsmittel in der Kosmetik. Da DHA wasserlöslich ist, stellen übliche Hautbräunungsmittel stets O/W- oder W/O-Emulsionen dar, bei denen DHA in der Wasserphase gelöst ist und die Verbindung mit der Ölphase durch einen Emulgator hergestellt wird. Deutlich schwieriger und bisher noch nicht bekannt ist die Einbeziehung von DHA in pastenartige Produkte auf Öl- und Wachsbasis. Auch in solchen Produkten sind Wassergehalte von wenigstens 5-10% erwünscht, um einen gewissen Frische-Effekt zu ermöglichen. Höhere Wachs- und Ölanteile, die zu pastenartigen Konsistenzen führen, erfordern jedoch entweder entsprechende Emulgatoren oder führen zu wenig lagerstabilen Formulierungen. Wegen möglicher Hautirritationen ist jedoch der Einsatz von Emulgatoren in der letzten Zeit sehr kritisch betrachtet worden, so dass emulgatorfreie Produkte mehr und mehr bevorzugt werden.

EP-1 473 026 und EP-1 477 159 offenbaren emulgatorfreie kosmetische Zubereitungen zur Hauttönung, die Dihydroxyaceton, Wasser, Wachse, Öle und Pigmente enthalten.

Der Erfindung liegt die Aufgabe zugrunde, neue Hauttönungsmittel mit DHA zu entwickeln, die emulgatorfrei sind und Wasser, Wachs und Öl enthalten.

Eine weitere Aufgabe besteht in der Bereitstellung eines Hauttönungsmittels mit unmittelbar nach dem Auftragen auf die Haut entstehenden Tönungseffekt.

Eine weitere Aufgabe besteht darin, ein Hauttönungsmittel mit deutlichem Frische-Effekt bereitzustellen.

Die Aufgaben werden gelöst durch ein neues Hauttönungsmittel, das folgende Bestandteile umfasst

| | |
|---|---|
| Dihydroxyaceton | 1 bis 3 Gewichts-% |
| Wasser | 8 bis 35 Gewichts-% |
| Wachse | 5 bis 30 Gewichts-% |
| Öle | 3 bis 35 Gewichts-% |
| Pigmente | 1 bis 7 Gewichts-% |

und weitere kosmetische Hilfsstoffe, Trägerstoffe oder Gemische davon mit Ausnahme von Emulgatoren für die Emulgierung von Wasser- und Ölphase, und wobei das Verhältnis von Wachsen und Ölen im Bereich von 0,8 bis 1 : 1,8 bis 2,3 liegt,
und das Hauttönungsmittel bei Raumtemperatur eine feste Masse bildet.

Das erfindungsgemäße Hauttönungsmittel ruft keine kräftige Hautfärbung, sondern eine dezente Hauttönung hervor, weshalb der DHA-Anteil auf 3 Gew.-% begrenzt ist. Vorzugsweise liegt der Anteil an DHA im Bereich von 1 bis 2,5 Gew.-%, besonders bevorzugt im Bereich von 1 bis 2 Gew.-%, bezogen auf das Gesamtgewicht des Hauttönungsmittels. DHA liegt als freies wasserlösliches DHA in dem erfindungsgemäßen Hauttönungsmittel vor. Auf aufwendige DHA-Trägersysteme, wie beispielsweise Liposomen, Lipidkapseln oder lamellare Strukturen kann verzichtet werden.

Eine lagerstabile kosmetische Formel mit wasserlöslichem DHA lässt sich jedoch nur erreichen, wenn die Höchstgehalte der anderen Wirkbestandteile Wasser, Wachse und Öle nicht überschritten werden und das Verhältnis Wachse zu Öle eingehalten wird.

Der Gesamtwassergehalt beträgt höchstens 35 Gew.-%.

Der Höchstbestandteil an nicht gebundenem Wasser für die Ausbildung der wässrigen Phase, die mit der Ölphase vermischt wird, beträgt 30 Gew.-%. Es können bis 5 Gew.-% zusätzliche Wasseranteile enthalten sein, die in Form von Wasser vorliegen, das an andere Ingredienzien gebunden ist. Dies können z.B. Liposome oder wässrige bzw. wässrig-alkoholische Pflanzenextrakte sein. Ein bevorzugter Wassergehalt an nicht gebundenem Wasser liegt bei 10-28 Gew.-%, insbesondere 15-25 Gew.-%, besonders bevorzugt 21-24 Gew-%.

Das Verhältnis von Wachsen und Ölen ist so einzustellen, dass prozentual nicht mehr Wachse als Öle vorliegen. Das Verhältnis Wachs:Öl ist 0,8-1 : 1,8-2,3.

Das Hauttönungsmittel bildet bei Raumtemperatur eine feste Masse, ähnlich wie eine Lippenstiftmasse, so dass eine Viskositätsangabe nicht möglich ist. Das Mittel erweicht bei höherer Temperatur von 30 bis 57 °C und hat einen Schmelzpunkt im Bereich von 58-75 °C. Unter Raumtemperatur wird der Temperaturbereich von 18-25 °C verstanden. Die ausgewählten Wachse und Öle sowie deren Verhältnis zueinander haben Einfluß auf den Schmelzpunkt in dem genannten Bereich.

Das erfindungsgemäße matte, nichtglänzende Hauttönungsmittel, das nach dem Abfüllen als feste, rissfreie Masse vorliegt, zeigt eine sofortige und gleichmäßige Hauttönung über mehrere Tage mit einem komfortablen Hautgefühl, zeigt weiterhin einen ausgezeichneten Frische-Effekt und ist lagerstabil über wenigstens 24 Monate.

Das erfindungsgemäße Mittel bildet überraschenderweise ein homogenes Gemisch der Basisbestandteile Wasser-Wachs-Öl, auch ohne Anwesenheit von Emulgatoren, und zeigt in dieser Form auch eine sehr gute Lagerstabilität von wenigstens 24 Monaten, vorzugsweise wenigstens 36 Monaten.

Im Unterschied zu anderen wachs- und ölhaltigen Produkten weist es einen hohen Wassergehalt auf und kann auf Emulgierhilfen verzichten. Dadurch wird erfindungsgemäß ein Hauttönungsmittel bereit gestellt, dass auch für empfindliche Haut gut geeignet ist und nicht irritierend wirkt. Auf Grund des vorhandenen Wassers wird neben der Hauttönung auch eine Hautpflege, insbesondere eine Verbesserung der Hautfeuchtigkeit, erreicht.

Unter Öle im Sinne der Erfindung werden übliche kosmetische Öle verstanden sowie auch kosmetische Ester und Ether. Vorzugsweise werden die Öle ausgewählt unter einem oder mehreren Siliconölen, einem oder mehreren pflanzlichen Ölen oder einem oder mehreren Fettsäureestern oder Gemischen davon.

Besonders geeignete Öle sind beispielsweise Siliconöle, Mineralöle, Hydrogenated Polyisobuten, Polyisopren, Squalane, Tridecyltrimellitat, Trimethylpropan-triisostearat, Isodecylcitrat, Neopentylglycol-diheptanoat, PPG-15-stearylether sowie pflanzliche Öle, wie z.B. Calendulaöl, Jojobaöl, Avocadoöl, Macadamianussöl, Rizinusöl, Kakaobutter, Shea-Butter, Kokosnussöl, Maisöl, Baumwollsamenöl, Olivenöl, Palmkernöl, Rapssamenöl, Safloröl, Sesamsamenöl, Sojabohnenöl, Sonnenblumensamenöl, Weizenkeimöl, Traubenkernöl oder Distelöl und Gemische davon.

Besonders bevorzugt sind Siliconöle und pflanzliche Öle. Bevorzugte Siliconöle sind polymerisierte Siliconöle, insbesondere polymerisiertes Cyclopentasiloxan, Cyclohexasiloxan oder Dimethylsiloxan und Gemische dieser polymerisierten Siloxane. Erfindungsgemäß bevorzugt sind ein Gemisch aus Cyclopentasiloxan und Cyclohexasiloxan, beispielsweise 65 % Cyclopentasiloxan und 35% Cyclohexasiloxan (erhältlich z.B. als Silicone DC 345 (Dow Corning)) oder Polyethermodifizierte Siliconöle, wie z.B. PEG-10 Dimethicone & PEG-10 (erhältlich z.B. als polyether modified silicone fluid KF6017 (Shin-Etsu Silicones of America)). Ein besonders bevorzugtes Pflanzenöl ist Jojobaöl.

Erfindungsgemäß als Öle verwendete kosmetische Ester oder Ether sind zum Beispiel geeignet Dipentaerythrityl Hexacaprylate/ Hexacaprate, Tridecyl Trimellitate/Tridecyl Stearate, Neopentyl Glycol Dicaprylate, Propylene Glycol Dioctanoate, Propylene Glycol Dicaprylate-2,30-Dicaprate, Dibutyl Adipate, Tridecyl Stearate/Neopentyl Glycol Dicaprylate Dicaprate, Tridecyl Trimellitate, Neopentyl Glycol Dioctanoate, Isopropyl Myristate, Diisopropyl Dimer Dilinoleate, , Myristyl Ether, Stearyl Ether, Cetearyl Octanoate, Butyl Ether, Dicaprylyl Ether, PPG15 Stearyl Ether, PPG14 Butyl Ether, Fomblin HC25, oder Gemische davon. Besonders geeignet ist beispielsweise Dibutyl Adipate.

Der bevorzugte Anteil an Ölen liegt im Bereich von 15 bis 35 Gew.-%, besonders bevorzugt 25 bis 35 Gew.-%, bezogen auf die Gesamtzusammensetzung des Hauttönungsmittels.

Die erfindungsgemäß eingesetzten Wachse können ausgewählt werden unter natürlichen pflanzlichen Wachsen, tierischen Wachsen, natürlichen und synthetischen Mineralwachsen und synthetischen Wachsen. Dazu gehören beispielsweise Carnaubawachs, Candellilawachs, Ozokerit, Montanwachs, Ceresin, Mikrowachse, Hartparaffin, Petrolatum, Siliconwachs oder nicht-emulgierende Polyethylenglycol- oder -glycolesterwachse und Gemische davon. Besonders geeignet sind Ceresin, mikrokristalliner Wachs, Carnaubawachs, Ozokerit oder Siliconwachse wie z.B. Stearyl Dimethicone und Gemische davon.

Der bevorzugte Gehalt an Wachsen liegt im Bereich von 8 bis 24 Gew.-%, besonders bevorzugt 12 bis 22 Gew-%, bezogen auf die Gesamtzusammensetzung des Hauttönungsmittels.

Pigmente, Pigmentgemische oder Pulver mit pigmentartiger Wirkung, worunter auch solche mit Perlglanz- oder Farbeffekt zu verstehen sind, können zum Beispiel umfassen Eisenoxide, natürliche Aluminiumsilicate wie z.B. Ocker, Titandioxid, Glimmer, Kaolin, manganhaltige Tone, Zinkoxid, Calciumcarbonat, Talkum, Glimmer-Titanoxid, Glimmer-Titanoxid-Eisenoxid, Nylonkügelchen, Keramikkügelchen, expandierte und nichtexpandierte synthetische Polymerpulver, pulverförmige natürliche organische Verbindungen wie z.B. gemahlene Festalgen, gemahlene Pflanzenteile oder verkapselte und unverkapselte Getreidestärken und Gemische davon. Besonders bevorzugt sind Eisenoxide, Titandioxid oder Kaolin und Gemische davon.

Bevorzugte Bereiche für den Pigmentanteil liegen bei 0,5 bis 5 Gew.-%, insbesondere 0,7 bis 2 Gew.-%, bezogen auf die Gesamtzusammensetzung.

Da Pigmente eine Sofortfärbung der Haut bewirken, und die Färbung durch DHA etwas langsamer aber dafür über einen längeren Zeitraum eintritt, ergibt sich innerhalb der angegebenen Mengen, insbesondere in den Vorzugsbereichen, mit den bevorzugten Pigmenten eine sofortige Hauttönung in einem sehr natürlichen Hautton, die auch über einen längeren Zeitraum von mehreren Tagen anhält.

Das erfindungsgemäße Mittel enthält weiterhin kosmetische Hilfs- und Trägerstoffe, wie sie üblicherweise in solchen Zubereitungen verwendet werden, z.B. Konservierungsmittel, Farbstoffe, Verdickungsmittel, Duftstoffe, Alkohole, Polyole, Elektrolyte, Gelbildner, Polymere, wie z.B. Polyethylene, beispielsweise erhältlich als Performalene 400 (New Phase Technologies), Copolymere, wie z.B. Methyl Methacrylate Crosspolymer, beispielsweise erhältlich als Covabead LH85 (Goldman), Stabilisatoren, Antioxidationsmittel oder Füllstoffe wie z.B. Silica, Talkum, Nylon, vorzugsweise Nylon-12, beispielsweise erhältlich als Orgasol 2002 EXD NAT COS (Arkema) oder Magnesiumsulfat und Gemische davon.

Geeignete Polyole sind z.B. Propylenglycol, Dipropylenglycol, Ethylenglycol, Isoprenglycol, Glycerin, Butylenglycol oder Sorbitol und Gemische davon. Der Anteil des Polyols liegt im Bereich von 0,5 bis 20 Gew.-%, vorzugsweise von etwa 1% bis etwa 5 Gew-% der Gesamtzusammensetzung.

Zu Antioxidationsmitteln gehören Vitamine wie z.B. Vitamin C und Derivate davon, beispielsweise Ascorbylacetat, -phosphat und -palmitat, Magnesiumascorbylphosphat; Vitamin A und Derivate davon; Folsäure und deren Derivate; Vitamin E und deren Derivate, wie z.B. Tocopherylacetat; Flavone oder Flavonoide; Aminosäuren, wie z.B. Histidin, Glycin, Tyrosin, Tryptophan und Derivate davon; Imidazole wie z.B. cis- oder trans-Urocaninsäure und deren Derivate; Peptide wie z.B. D,L-Carnosin, D-Carnosin, L-Carnosin und deren Derivate; Carotinoide und Carotine, wie z.B α-Carotin, β-Carotin, Lycopin; Harnsäure und Derivate davon; α-Hydroxysäuren wie z.B. Citronensäure, Milchsäure, Apfelsäure; α-Hydroxyfettsäuren wie z.B. Phytinsäure, Lactoferrin; Stilbene und deren Derivate; Mannose und deren Derivate; Liponsäure und deren Derivate, z.B. Dihydroliponsäure; Ferulasäure und deren Derivate; Thiole wie z.B. Glutathion, Cystein, Cystin und deren Ester oder Pflanzenbestandteile oder -extrakte und Gemische davon.

Ein besonders bevorzugtes Antioxidationsmittel ist eine Wirkstoffzubereitung mit Pflanzenextrakten, umfassend 0,1 bis 2 Gew-% Extrakt grüner Kaffeebohnen (INCI: Coffea arabica (coffee) seed extract, CAS number 84650-00-0), 0,1 bis 2 Gew-% Extrakt von Blättern von Camellia sinensis (INCI: Camellia sinensis Leaf Extract, CAS number 84650-60-2), 0,1 bis 2 Gew-% Extrakt von Pongamia pinnata (INCI: Pongamia pinnata seed extract) und 0,1 bis 2 Gew-% Extrakt der Wurzeln von Angelica archangelica (INCI: Angelica archangelica root extract, CAS number 84775-41-7) und wenigstens einen einwertigen C₂-C₅-Alkohol (7-10 Gew.-%) und gegebenenfalls weitere Hilfsstoffe. Die Pflanzenextrakte werden vorzugsweise zu gleichen Teilen miteinander vermischt.

In einer besonders bevorzugten Ausführungsform wird ein in Lecithin verkapseltes Gemisch aus (in Gew.- %) 2 % Angelica archangelica-Wurzelextrakt, 2 % Pongamia pinnata-Samenextrakt, 2 % Camelia sinensis-Blattextrakt, 2 % Coffea arabica-Samenextrakt verwendet, wobei außerdem 8 % Glycerin, 8,25 % Alkohol denaturiert, Antioxidationsmittel und Hilfsstoffe enthalten sind.

Weitere bevorzugte Antioxidationsmittel sind Tocopherol, Ascorbinsäure, , Extrakte der Echten Vanille (Vanilla planifolia), wie z.B. Vanilla planifolia Extract & Water & Propylene Glycol, beispielsweise erhältlich als Vanille Extrakt D.C. (4013) (Provital Group), Veilchenextrakt (Viola tricolor), wie z.B. Viola Tricolor Extract & Water, beispielsweise erhältlich als Aquaphyline (Silab), oder Mönchspfeffer-Extrakt (Vitex agnus castus), wie z.B. Vitex agnus castus Extract & Water & Glycerine & Alcohol, beispielsweise erhältlich als Happybelle-PE (Mibelle AG Biochemistry) und Gemische davon. Besonders bevorzugt ist auch ein Gemisch aus (a) einem Produkt, erhalten durch Extraktion der Rinde von Quebracho blanco und nachfolgender enzymatischer Hydrolyse und verkapselt in Mikrokapseln, (b) einem durch Extraktion gewonnenen Seidenraupenextrakt, (c) einem Hydro-Gel oder Gemisch von Hydro-Gelen, (d) einem oder mehreren Phospholipiden und (e) Wasser gemäß WO99/66881, Anspruch 1.

Der Gehalt an Antioxidationsmitteln liegt vorteilhaft im Bereich von 0,05 bis 4 Gew.-%, insbesondere 0,1 bis 1 Gew.-% der Wirkstoffe, bezogen auf die Gesamtzusammensetzung.

Das erfindungsgemäße Mittel kann auch übliche Feuchthaltemittel enthalten, wie z.B. Glycerin, Butylenglycol, Propylenglycol, Hyaluronsäure, Lactose, Glycin, Allantoin oder Sorbitol und Gemische davon.

Neben DHA kann das Hauttönungsmittel der Erfindung weitere färbende Bestandteile enthalten, wie z.B. Isatin, Alloxan, Ninhydrin, Glycerinaldehyd, meso-Weinsäurealdehyd, Glutaraldehyd, Erythrulose, Pirazolin-4,5-dionderivate, 4,4-Dihydroxypirazolin-5-dionderivate, Glycyrrhiza glabra (Süßholzwurzel).

Das erfindungsgemäße Hauttönungsmittel kann weiterhin ein Sauerstoff-Trägersystem aus mit Sauerstoff beladenen asymmetrischen lamellaren Aggregaten gemäß WO 94/00109, Anspruch 1 enthalten oder ein Sauerstoff-Trägersystem aus einem flüssigen perfluorierten oder teilfluorierten Kohlenwasserstoff oder Kohlenwasserstoffgemisch mit einem Anteil von 1-10 Gew-% bezogen auf das Gesamtgewicht der Formulierung, einem flüssigen Siliconpolymeren oder Siliconpolymerengemisch und einer Öl- oder Wasserbasis enthalten, wobei das Trägersystem mit gasförmigem Sauerstoff bis zu einem Partialdruck von 150-950 mbar O₂ beladen ist gemäß WO 05/053636, Ansprüche 1 und 2.

Ein weiterer Hilfsstoff, den das erfindungsgemäße Mittel enthalten kann, ist eine Suspension, die fein verteilte hartmagnetische Einbereichsteilchen (Einkristalle) mit hoher Koerzitivfeldstärke von 3000 bis 5000 Oerstedt und mit Korngrößen im Bereich von 80 bis 1200 nm, vorzugsweise 80-250 nm enthält, wobei diese hartmagnetischen Teilchen insbesondere Barium- und/oder Strontiumhexaferrite sind, die nach der Glaskristallisationstechnik durch Züchtung von Einkristallen aus einer abgeschreckten Glasschmelze erzeugt werden. Geeignete Suspensionen sind beispielweise in der WO95/03061 z.B. Beispiel 2 oder 3 oder der WO98/44895 z.B. Beispiel 1C beschrieben. Die hartmagnetischen Teilchen können auch mit den o.g. Sauerstoff-Trägersystemen kombiniert werden.

In einer bevorzugten Ausführungsform der Erfindung weist das erfindungsgemäße Hauttönungsmittel mindestens alle Merkmale des unabhängigen Anspruchs 1 sowie eines abhängigen Anspruchs auf. In einer weiter bevorzugten Ausführungsform weist das erfindungsgemäße Hauttönungsmittel mindestens die Merkmale des unabhängigen Anspruchs 1 und eine Kombination der Merkmale aus zwei oder mehreren abhängigen Ansprüchen auf.

In einer besonders bevorzugten Ausführungsform enthält das erfindungsgemäße Hauttönungsmittel neben Dihydroxyaceton, Wasser, Wachsen, Ölen und Pigmenten, noch Polymere, viskositätsregulierende Mittel und Antioxidationsmittel sowie gegebenenfalls Feuchthaltemittel, Haut conditioner, Füllstoffe, Konservierungsmittel oder Gemische davon.

Die Erfindung betrifft auch ein Verfahren zur Herstellung eines kosmetischen Hauttönungsmittels, bei dem man eine homogene Wasserphase, umfassend Dihydroxyaceton und andere wasserlösliche kosmetische Hilfsstoffe, bei einer Temperatur im Bereich von 40 bis 60°C, vorzugsweise von 50 bis 60°C unter Rühren herstellt, und
eine homogene Ölphase, umfassend Öle, Wachse und Pigmente, bei einer Temperatur im Bereich von 75 bis 88°C, vorzugsweise von 80 bis 85°C herstellt,
die Wasserphase der Ölphase unter Rühren zusetzt, wobei die Temperatur bei der Zugabe im Bereich von 68 bis 75°C liegt,
das erhaltene Gemisch auf Raumtemperatur abkühlt, wobei die Masse erstarrt, die abgekühlte Masse auf 68 bis 70°C wieder erwärmt,
die erwärmte Masse in Formen gießt und die gefüllten Formen in einem Zeitraum von 1 bis 10 Minuten, vorzugsweise von 6 bis 10 Minuten, noch bevorzugter, innerhalb von 8 bis 10 Minuten auf Umgebungstemperatur abkühlt. Die Abkühlung kann vorteilhaft in einem Kühltunnel bei 4 bis 5°C erfolgen. Die resultierende Masse ist in ihrer Konsistenz mit einer erstarrten Lippenstiftmasse vergleichbar.

Die Erfindung betrifft ferner die Verwendung des erfindungsgemäßen Hauttönungsmittels zur kosmetischen Tönung der Haut, vorzugsweise der Gesichtshaut. Das erfindungsgemäße Hauttönungsmittel kann weiterhin verwendet werden, um Hautunreinheiten, Hautunebenheiten, Falten und/oder eine ungleichmäßige Pigmentierung zu egalisieren und/oder zu verdecken. Vorteilhafterweise kann das erfindungsgemäße Hauttönungsmittel auch als Hautpflegemittel, insbesondere zur Regulation bzw. zum Erhalt der Feuchtigkeit in der Haut, verwendet werden.

Die Erfindung soll nachstehend durch Beispiele näher erläutert werden. Alle Angaben erfolgen in Gewichtsprozent, sofern nichts anderes angegeben ist.

### Beispiel1 Kompakt-Tönungsmittel I

| **Phase A** | |
|---|---|
| Wasser | q.s. ad 100 |
| Dihydroxyaceton | 1,7 |
| Viola Tricolor Extract & Water | 1,3 |
| Vanilla planifolia Extract & Water & Propylene Glycol | 1,2 |
| Glycerine | 3,5 |
| Polyethylene | 1,2 |
| Vitex agnus castus Extract & Water & Glycerine & Alcohol | 1,1 |
| Nylon | 4,0 |
| RPF-Komplex * | 0,4 |
| Lauryl PEG-9 Polydimethylsiloxyxyethyl Dimethicone | 0,6 |

| **Phase B** | |
|---|---|
| Ceresin | 3,2 |
| Mikrokristallines Wachs | 1,8 |
| Dibutyl Adipate | 2,3 |
| PEG-10 Dimethicone & PEG-10 | 1,3 |
| Cyclopentasiloxane & Cyclohexasiloxane | 26 |
| Butylene Glycol | 1,8 |
| Talkum | 2,5 |
| Titandioxid | 1,9 |
| Eisenoxidpigmente | 1,0 |
| Phenoxyethanol | 0,7 |
| Carnauba Wax | 2,8 |
| Ozokerit | 4,1 |
| Stearyl Dimethicone | 2,4 |
| Silica | 2,8 |
| Magnesium Sulfate | 1,0 |
| Methyl Methacrylate Crosspolymer | 2,7 |

| | |
|---|---|
| RPF-Komplex * = Extraktgemisch aus grüner Kaffeebohne, Camellia sinensis, Pongamia pinnata und Angelica archangelica (7-10% des Extraktgemisches), Ethanol, Wasser (70%), Glycerin, Lecithin, Ascorbylpalmitat, Tocopherol(0,2%), PEG-8 (0,2%), Ascorbinsäure (0,2%) und Konservierungsmittel (1 %). | |

Die Bestandteile der Phase A werden nacheinander zusammengegeben und bei 40-50 °C gerührt. Die Bestandteile der Phase B werden nacheinander zusammengegeben und unter Rühren auf ca. 80-85 °C erhitzt. Zur Phase B wird Phase A unter Rühren bei einer Temperatur von 70 °C zugegeben. Nach dem Abkühlen bis zum Erreichen einer festen Konsistenz wird die Masse erneut unter Rühren auf 68 °C erwärmt und bei dieser Temperatur in Glas- oder Plastikbehälter abgefüllt und dann innerhalb von 8-10 Minuten auf Umgebungstemperatur abgekühlt. Man erhält eine feste Masse ähnlich wie eine Lippenstiftmasse.

### Beispiel 2 Kompakt-Tönungsmittel II

| **Phase A** | |
|---|---|
| Wasser | q.s. ad 100 |
| Dihydroxyaceton | 1,2 |
| Viola Tricolor Extract & Water | 1,5 |
| Vanilla planifolia Extract & Water & Propylene Glycol | 1,4 |
| Glycerine | 3,8 |
| Polyethylene | 1,0 |
| Vitex agnus castus Extract & Water & Glycerine & Alcohol | 1,5 |
| Nylon | 3,8 |
| RPF-Komplex * | 0,2 |
| Lauryl PEG-9 Polydimethylsiloxyxyethyl Dimethicone | 0,6 |
| **Phase B** | |
| Ceresin | 1,8 |
| Mikrokristallines Wachs | 2,0 |
| Dibutyl Adipate | 2,4 |
| PEG-10 Dimethicone & PEG-10 | 1,3 |
| Cyclopentasiloxane & Cyclohexasiloxane | 22 |
| Jojoba Oil | 5,0 |
| Butylene Glycol | 1,2 |
| Talkum | 2,8 |
| Parfüm | 1,0 |
| Titandioxid | 2,3 |
| Eisenoxidpigmente | 1,8 |
| Phenoxyethanol | 0,7 |
| Carnauba Wax | 4,0 |
| Ozokerit | 4,9 |
| Stearyl Dimethicone | 2,7 |
| Silica | 2,1 |
| Magnesium Sulfate | 1,2 |
| Methyl Methacrylate Crosspolymer | 2,7 |

| | |
|---|---|
| (RPF-Komplex * vgl. Beispiel 1) | |

Die Herstellung erfolgt wie im Beispiel 1, die Verarbeitung (Gießen) bei 69°C. Man erhält eine feste Masse wie im Beispiel 1.

### Beispiel 3 Kompakt-Tönungsmittel III

| **Phase A** | |
|---|---|
| Wasser | q.s. ad 100 |
| Dihydroxyaceton | 1,7 |
| Viola Tricolor Extract & Water | 1,3 |
| Vanilla planifolia Extract & Water & Propylene Glycol | 1,2 |
| Glycerine | 3,5 |
| Polyethylene | 1,2 |
| Vitex agnus castus Extract & Water & Glycerine & Alcohol | 1,1 |
| Nylon | 4,0 |
| RPF-Komplex * | 0,4 |
| Lauryl Aminopropylglycine | 0,6 |

| **Phase B** | |
|---|---|
| Ceresin | 3,2 |
| Mikrokristallines Wachs | 1,8 |
| Dibutyl Adipate | 2,3 |
| PEG-10 Dimethicone & PEG-10 | 1,3 |
| Cyclopentasiloxane & Cyclohexasiloxane | 26 |
| Butylene Glycol | 1,8 |
| Talkum | 2,5 |
| Titandioxid | 1,9 |
| Eisenoxidpigmente | 1,0 |
| Phenoxyethanol | 0,7 |
| Carnauba Wax | 2,8 |
| Ozokerit | 4,1 |
| Stearyl Dimethicone | 2,4 |
| Silica | 2,8 |
| Magnesium Sulfate | 1,0 |
| Methyl Methacrylate Crosspolymer | 2,7 |
| (RPF*-Komplex vgl. Beispiel 1) | |

Die Herstellung erfolgt wie im Beispiel 1, die Verarbeitung (Gießen) bei 68°C. Man erhält eine feste Masse wie im Beispiel 1.

### Beispiel 4 Kompakt-Tönungsmittel IV

| **Phase A** | |
|---|---|
| Wasser | q.s. ad 100 |
| Dihydroxyaceton | 1,2 |
| Viola Tricolor Extract & Water | 1,5 |
| Vanilla planifolia Extract & Water & Propylene Glycol | 1,4 |
| Glycerine | 3,8 |
| Polyethylene | 1,0 |
| Vitex agnus castus Extract & Water & Glycerine & Alcohol | 1,5 |
| Nylon | 3,8 |
| RPF-Komplex * | 0,2 |
| Lauryl Aminopropylglycine | 0,6 |
| **Phase B** | |
| Ceresin | 1,8 |
| Mikrokristallines Wachs | 2,0 |
| Dibutyl Adipate | 2,4 |
| PEG-10 Dimethicone & PEG-10 | 1,3 |
| Cyclopentasiloxane & Cyclohexasiloxane | 22 |
| Jojoba Oil | 5,0 |
| Butylene Glycol | 1,2 |
| Talkum | 2,8 |
| Parfüm | 1,0 |
| Titandioxid | 2,3 |
| Eisenoxidpigmente | 1,8 |
| Phenoxyethanol | 0,7 |
| Carnauba Wax | 4,0 |
| Ozokerit | 4,9 |
| Stearyl Dimethicone | 2,7 |
| Silica | 2,1 |
| Magnesium Sulfate | 1,2 |
| Methyl Methacrylate Crosspolymer | 2,7 |
| (RPF-Komplex * vgl. Beispiel 1) | |

Die Herstellung erfolgt wie im Beispiel 1, die Verarbeitung (Gießen) bei 69°C. Man erhält eine feste Masse wie im Beispiel 1.

## Patentansprüche

1. Kosmetisches Hauttönungsmittel, **dadurch gekennzeichnet, dass** es folgende Bestandteile umfasst
| | |
|---|---|
| Dihydroxyaceton | 1 bis 3 Gewichts-% |
| Wasser | 8 bis 35 Gewichts-% |
| Wachse | 5 bis 30 Gewichts-% |
| Öle | 3 bis 35 Gewichts-% |
| Pigmente | 1 bis 7 Gewichts-% |
und weitere kosmetische Hilfsstoffe, Trägerstoffe und Gemische davon mit Ausnahme von Emulgatoren für die Emulgierung von Wasser- und Ölphase,
und wobei das Verhältnis von Wachsen und Ölen im Bereich von 0,8 bis 1 : 1,8 bis 2,3 liegt,
und das Hauttönungsmittel bei Raumtemperatur eine feste Masse bildet, und
alle Gewichtsangaben auf das Gesamtgewicht des Mittels bezogen sind.

2. Hauttönungsmittel nach Anspruch 1, **dadurch gekennzeichnet, dass** der Wassergehalt im Bereich von 10-28 Gew.-%, insbesondere 15-25 Gew.-%, besonders bevorzugt 21-24 Gew-% liegt.

3. Hauttönungsmittel nach Anspruch 1, **dadurch gekennzeichnet, dass** der Gehalt an Wachsen im Bereich von 8 bis 24 Gew.-%, besonders bevorzugt 12 bis 22 Gew-% liegt.

4. Hauttönungsmittel nach Anspruch 1, **dadurch gekennzeichnet, dass** das Wachs Ceresin, mikrokristallines Wachs, Carnaubawachs, Ozokerit, Siliconwachs, vorzugsweise Stearyl Dimethicone, oder ein Gemisch davon ist.

5. Hauttönungsmittel nach Anspruch 1, **dadurch gekennzeichnet, dass** der Pigmentanteil im Bereich von 0,5 bis 5 Gew.-%, insbesondere 0,7 bis 2 Gew.-% liegt.

6. Hauttönungsmittel nach Anspruch 1, **dadurch gekennzeichnet, dass** das Öl ausgewählt ist unter einem oder mehreren Siliconölen, einem oder mehreren pflanzlichen Ölen, einem oder mehreren Fettsäureestern und Gemischen davon.

7. Hauttönungsmittel nach Anspruch 1, **dadurch gekennzeichnet, dass** das Öl ein polymerisiertes Siliconöl ist, insbesondere polymerisiertes Cyclopentasiloxan, Cyclohexasiloxan, Dimethylsiloxan und Gemische davon.

8. Hauttönungsmittel nach Anspruch 1, **dadurch gekennzeichnet, dass** der Gehalt an Ölen im Bereich von 15 bis 35 Gew.-%, besonders bevorzugt im Bereich von 25 bis 35 Gew-% liegt.

9. Verfahren zur Herstellung eines kosmetischen Hauttönungsmittels nach Anspruch 1, **dadurch gekennzeichnet, dass** man eine homogene Wasserphase, umfassend Dihydroxyaceton und andere wasserlösliche kosmetische Hilfsstoffe, bei einer Temperatur im Bereich von 50 bis 60°C unter Rühren herstellt, und
eine homogene Ölphase, umfassend Öle, Wachse und Pigmente, bei einer Temperatur im Bereich von 75 bis 88 °C herstellt,
die Wasserphase der Ölphase unter Rühren zusetzt, wobei die Temperatur bei der Zugabe im Bereich von 68 bis 75 °C liegt,
das erhaltene Gemisch auf 18 bis 25°C abkühlt,
die abgekühlte Masse auf 68 bis 70°C wieder erwärmt,
die erwärmte Masse in Formen gießt und
die gefüllten Formen in einem Zeitraum von 6 bis 10 Minuten auf Umgebungstemperatur abkühlt.

10. Verwendung eines Hauttönungsmittels nach einem der Ansprüche 1 bis 8 zur kosmetischen Tönung der Haut, vorzugsweise der Gesichtshaut.

11. Verwendung eines Hauttönungsmittels nach einem der Ansprüche 1 bis 8 zur Verdeckung und/oder Egalisierung von Hautunreinheiten, Hautunebenheiten, Falten und/oder einer ungleichmäßigen Pigmentierung.

12. Verwendung eines Hauttönungsmittels nach einem der Ansprüche 1 bis 8 zur Hautpflege, vorzugsweise zur Feuchtigkeitsregulation der Haut.

## Claims

1. A cosmetic skin tanning product, **characterized in that** it comprises the following ingredients:
| | |
|---|---|
| Dihydroxyacetone | 1 to 3 % by weight |
| Water | 8 to 35 % by weight |
| Waxes | 5 to 30 % by weight |
| Oils | 3 to 35 % by weight |
| Pigments | 1 to 7 % by weight |
and further cosmetic auxiliaries, carriers, and mixtures thereof with the exception of emulsifiers for emulsifying the water and oil phases,
and wherein the ratio of waxes to oils is in the range of 0.8 to 1 : 1.8 to 2.3,
and the skin tanning product is a solid compound at room temperature, and all weight percentages are relative to the total weight of the product.

2. A skin tanning product according to claim 1, **characterized in that** the water content is in the range of 10-28 % by weight, in particular 15-25 % by weight, particularly preferred 21-24 % by weight.

3. A skin tanning product according to claim 1, **characterized in that** the wax content is in the range of 8 to 24 % by weight, particularly preferred 12 to 22 % by weight.

4. A skin tanning product according to claim 1, **characterized in that** the wax is ceresin, microcrystalline wax, carnauba wax, ozokerite, silicone wax, preferably stearyl dimethicone, or a mixture thereof.

5. A skin tanning product according to claim 1, **characterized in that** the pigment content is in the range of 0.5 to 5 % by weight, in particular 0.7 to 2 % by weight.

6. A skin tanning product according to claim 1, **characterized in that** the oil is selected from among one or several silicone oils, one or several vegetable oils, one or several fatty acid esters, and mixtures thereof.

7. A skin tanning product according to claim 1, **characterized in that** the oil is a polymerized silicone oil, in particular polymerized cyclopentasiloxane, cyclohexasiloxane, dimethylsiloxane, and mixtures thereof.

8. A skin tanning product according to claim 1, **characterized in that** the oil content is in the range of 15 to 35 % by weight, particularly preferred in the range of 25 to 35 % by weight.

9. A method for producing a cosmetic skin tanning product according to claim 1, **characterized in that** a homogeneous water phase comprising dihydroxyacetone and other watersoluble cosmetic auxiliaries is prepared at a temperature ranging from 50 to 60°C while stirring, and
a homogeneous oil phase comprising oils, waxes, and pigments is prepared at a temperature ranging from 75 to 88°C,
the water phase is added to the oil phase while stirring, wherein the temperature ranges between 68 and 75°C during said addition,
the mixture obtained in this way is cooled down to 18 to 25°C,
the cooled-down compound is heated again to 68 to 70°C,
the heated compound is poured into moulds, and
the filled moulds are cooled down to ambient temperature during a period of time of 6 to 10 minutes.

10. The use of a skin tanning product according to any one of claims 1 to 8 for cosmetic tanning of the skin, preferably the facial skin.

11. The use of a skin tanning product according to any one of claims 1 to 8 to cover and/or smooth skin impurities, skin irregularities, wrinkles, and/or an uneven pigmentation.

12. The use of a skin tanning product according to any one of claim 1 to 8 for skin care, preferably to regulate the moisture content of the skin.

## Revendications

1. Agent cosmétique de teintage de la peau, **caractérisé en ce qu'**il comprend les composants suivante :
| | |
|---|---|
| dihydroxyacétone | 1 à 3 % en poids |
| eau | 8 à 35 % en poids |
| cire | 5 à 30 % en poids |
| huiles | 3 à 35 % en poids |
| pigments | 1 à 7 % en poids |
et d'autres adjuvants, véhicules cosmétiques et leurs mélanges à l'exception d'émulsifiants pour émulsifier des phases aqueuses et huileuses, et dans lequel le rapport des cires et huiles est de l'ordre de 0,8 à 1:1,8 à 2,3,
et **en ce que** l'agent de teintage de la peau forme une masse solide à température ambiante, toutes les données en poids se rapportant au poids total de l'agent.

2. Agent de teintage de la peau selon la revendication 1, **caractérisé en ce que** la teneur en eau est de l'ordre de 10 à 28 % en poids, en particulier, de 15 à 25 % en poids, de manière particulièrement préférée, de 21 à 24 % en poids.

3. Agent de teintage de la peau selon la revendication 1, **caractérisé en ce que** la teneur en cire est de l'ordre de 8 à 24 % en poids, de manière particulièrement préférée, de 12 à 22 % en poids.

4. Agent de teintage de la peau selon la revendication 1, **caractérisé en ce que** la cire est la cérésine, une cire microcristalline, la cire de carnauba, l'ozocérite, la cire de silicone, de préférence, la stéaryl-diméthicone ou leur mélange.

5. Agent de teintage de la peau selon la revendication 1, **caractérisé en ce que** la proportion de pigment est de l'ordre de 0,5 à 5 % en poids, en particulier de 0,7 à 2 % en poids.

6. Agent de teintage de la peau selon la revendication 1, **caractérisé en ce que** l'huile est choisie parmi une ou plusieurs huiles de silicone, une ou plusieurs huiles végétales, un ou plusieurs esters d'acide gras et leurs mélanges.

7. Agent de teintage de la peau selon la revendication 1, **caractérisé en ce que** l'huile est une huile de silicone polymérisée, en particulier, du cyclopentasiloxane polymérisé, du cyclohexasiloxane, du diméthylsiloxane et leurs mélanges.

8. Agent de teintage de la peau selon la revendication 1, **caractérisé en ce que** la teneur en huiles est de l'ordre de 15 à 35 % en poids, de manière particulièrement préférée, de l'ordre de 25 à 35 % en poids.

9. Procédé de production d'un agent cosmétique de teintage de la peau selon la revendication 1, **caractérisé en ce que** l'on produit une phase aqueuse homogène comprenant de la dihydroxyacétone et d'autres adjuvants cosmétiques hydrosolubles, à une température de l'ordre de 50 à 60° C en agitant, et
on produit une phase huileuse homogène comprenant des huiles, des cires et des pigments, à une température de l'ordre de 75 à 88° C,
on ajoute la phase aqueuse à la phase huileuse en agitant, la température se situant lors de l'addition dans une plage de 68 à 75° C,
le mélange obtenu est refroidi de 18 à 25° C,
la masse refroidie est réchauffée de 68 à 70° C,
la masse chauffée est coulée dans des moules et
les moules remplis sont refroidis en l'espace de 6 à 10 minutes à température ambiante.

10. Utilisation d'un agent de teintage de la peau selon l'une des revendications 1 à 8, pour le teintage cosmétique de la peau, de préférence de la peau du visage.

11. Utilisation d'un agent de teintage de la peau selon l'une des revendications 1 à 8, pour recouvrir et/ou uniformiser les impuretés cutanées, les inégalités cutanées, les rides et/ou une pigmentation non uniforme.

12. Utilisation d'un agent de teintage de la peau selon l'une des revendications 1 à 8, pour le soin de la peau, de préférence pour la régulation de l'hydratation de la peau.
